# EUROPÄISCHE PATENTSCHRIFT

(11) **EP 0 971 232 B1**
(45) Veröffentlichungstag und Bekanntmachung des Hinweises auf die Patenterteilung: **24.11.2004**
(21) Anmeldenummer: 99112802.6
(22) Anmeldetag: 02.07.1999
(51) Int. Cl.: G01N 33/50, C12Q 1/37, C12Q 1/34, C12Q 1/44, C12Q 1/66, C12N 5/00, C12N 9/96

(54) **Luminometrische ATP-Bestimmung**
Luminometric determination of ATP
Détermination luminométrique d'ATP

(30) Priorität: 05.07.1998 DE 19829893
(43) Veröffentlichungstag der Anmeldung: 12.01.2000
(73) Patentinhaber: Fraunhofer-Gesellschaft zur Förderung der angewandten Forschung e.V., 80686 München (DE)
(72) Erfinder: Mappes, Marion, Dr., 72131 Ofterdingen (DE)

(56) Entgegenhaltungen:
- WO-A-94/17177
- WO-A-94/24273
- WO-A-97/18296
- US-A- 5 242 806
- ROBIDOUX ET AL: "Release of Prostaglandin E2 and Thromboxane B2 by Mixed Isolated Human Lung Cells" PROSTAGLANDINS LEUKOTRIENES AND ESSENTIAL FATTY ACIDS, Bd. 32, 1988, Seiten 23-28, XP000909344
- WILLIAMSON K. ET AL: "In vitro BrdUrd incorporation of colorectal tumour tissue" CELL. PROLIF., Bd. 26, 1993, Seiten 115-124, XP000887087
- MUSHTAQ M. ET AL: "Gossypol inhibits human chorionic gonadotropin-stimulated testosterone production by cultured canine testicular interstitial cells" RESEARCH COMMUNICATIONS IN MOLECULAR PATHOLOGY AND PHARMACOLOGY, Bd. 91, 1996, Seiten 259-272, XP000909379
- KURBACHER C.M. ET AL: "Heterogeneity of in vitro chemosensitivity in perioperative breast cancer cells to mitoxantrone versus doxorubicin evaluated by a microplate ATP bioluminescence assay" BREAST CANCER RESEARCH AND TREATMENT, Bd. 41, 1996, Seiten 161-170, XP000887038
- SNEAD MARY D ET AL: "Isolation and culture of endothelial cells from the mesenteric vascular bed." METHODS IN CELL SCIENCE, Bd. 17, Nr. 4, 1995, Seiten 257-262, XP000973538 ISSN: 1381-5741
- YAU W M ET AL: "CHARACTERIZATION OF ACETYLCHOLINE RELEASE FROM ENZYME-DISSOCIATED MYENTERIC GANGLIA" AMERICAN JOURNAL OF PHYSIOLOGY, Bd. 256, Nr. 1 PART 1, 1989, Seiten G233-G239, XP000973354 ISSN: 0002-9513
- DEOME K B ET AL: "DETECTION OF INAPPARENT NODULE TRANSFORMED CELLS IN THE MAMMARY GLAND TISSUES OF VIRGIN FEMALE BALB-CFC-3H MICE" CANCER RESEARCH, Bd. 38, Nr. 7, 1978, Seiten 2103-2111, XP000945287 EN ISSN: 0008-5472
- REEL J R ET AL: "CYCLIC AND TEMPORAL DIFFERENCES IN LUTEINIZING HORMONE RELEASING HORMONE STIMULATED LUTEINIZING HORMONE RELEASE IN CULTURED RAT PITUITARY CELLS" MOLECULAR AND CELLULAR ENDOCRINOLOGY, Bd. 10, Nr. 2, 1978, Seiten 163-174, XP000973355 EN ISSN: 0303-7207
- RONG G H ET AL: "AN ENZYMATIC METHOD FOR THE CONSISTENT PRODUCTION OF MONODISPERSED VIABLE CELL SUSPENSIONS FROM HUMAN SOLID TUMORS" JOURNAL OF SURGICAL ONCOLOGY, Bd. 28, Nr. 2, 1985, Seiten 131-133, XP000945357 ISSN: 0022-4790
- MARCUS G J ET AL: "ENZYMATIC DISSOCIATION OF OVARIAN AND UTERINE TISSUES" ENDOCRINE RESEARCH, Bd. 10, Nr. 2, 1984, Seiten 151-162, XP000909317 ISSN: 0743-5800

## Beschreibung

Die vorliegende Erfindung betrifft ein Verfahren zum Bestimmen der Aktivität mindestens eines Cytostatikums gegenüber Tumormaterial, ein Verfahren zur luminometrischen ATP-Bestimmung von Tumormaterial sowie dabei verwendete Mittel.

Bekannte Therapien zur Bekämpfung von Krebskrankheiten umfassen häufig den Einsatz von Cytostatika. Die Wirkung dieser Cytostatika auf die Zielzellen, also die Tumorzellen, muß im Vorfeld der Therapie bestimmt werden. Dabei ist es besonders wünschenswert, die patientenspezifische Wirkung des einzusetzenden Cytostatikums zu kennen und entsprechende Testverfahren einzusetzen.

Während nämlich die Bestimmung der Sensitivität und der Resistenz von Mikroorganismen gegen Antibiotika seit vielen Jahren standardmäßig Patienten-bezogen durchgeführt wird, um eine optimale Behandlung von Infektionskrankheiten zu realisieren, ist hingegen die individuelle Patienten-bezogene Chemotherapie von Krebserkrankungen bisher nicht oder nur in Ansätzen verwirklicht. Die Anwendung von Cytostatikatests zur Entscheidung über die Wahl der Cytostatika beziehungsweise deren Kombination zur postoperativen Therapie hat sich bis heute jedoch nur ungenügend etabliert. Der Vorteil einer in-vitro Prädiagnostik liegt sowohl in der dadurch ermöglichten frühen Entscheidung für eine bestimmte Therapie als auch in der Verbesserung der Lebensqualität des Patienten und in der Einsparung von kostenintensiven Therapiesequenzen. Die Nachteile momentan vorliegender Tests begründen sich unter anderem auf einem sehr umfangreichen finanziellen und technischen Aufwand. Dieser Aufwand und die noch immer fragliche Übertragbarkeit der in-vitro-Ergebnisse auf die physiologische in-situ-Situation des Patienten lassen üblicherweise verwendete Cytostatikatests verbesserungswürdig erscheinen. Vergleichende Untersuchungen mit kommerziell erhältlichen Tests geben nur einen geringen Freiraum zur Weiterentwicklung und Anwendung im Bereich neuer Therapieansätze. Insbesondere ist es nur sehr begrenzt möglich, diese kommerziellen Tests auf die Untersuchung einzelner Patienten hin zu optimieren.

Die Zahl der Cytostatika und der daraus resultierenden Kombinationen steigt seit einigen Jahren kontinuierlich an, da die Natur und Heterogenität der unterschiedlichen Tumoren immer detaillierter aufgeschlüsselt werden kann. Die Entscheidung zu einem bestimmten Cytostatikum oder der aussichtsreichsten Kombination wird oftmals durch auftretende Non-Respons oder Resistenz des Patienten erschwert. Bei der Vielzahl der unterschiedlichen Parameter ist es schwierig, die richtige Auswahl an Parametern zu definieren, um optimale Aussagen über den Tumor und seine medikamentöse und/oder strahlentherapeutische Beeinflussung zu erhalten. Es wurde daher mehrfach der Versuch unternommen, Analysenmethoden zu entwickeln, die schnell und sicher zu einer Aussage führen. Zur Bestimmung der in-vitro-Wirksamkeit wurden zwei verschiedene Ansätze verfolgt. Aus der Vorstellung heraus, daß Tumoren monoclonalen oder oligoclonalen Ursprungs sind, wurden sogenannte clonogenic Assays entwikzelt, die jedoch an dem hohen Zeitaufwand scheiterten, der notwendig ist, um aus dem ursprünglichen Tumormaterial eine definierte Zellpopulation aus Clonen zu gewinnen. Die Evaluierung, das heißt das Verhältnis angewachsener Proben zur Gesamtzahl, zeigte große Varianzen, wodurch diese Form der Analyse schwer zu interpretieren ist (Hanauske und Hoff, 1986). Der zweite Ansatz umfaßt nonclonogenic Assays, die die Lebensfähigkeit und das Wachstum der Zellen messen. Hier tritt allerdings ein technisches Problem auf, denn bei direkter Zellaussaat kann das Überwachsen von nicht-entarteten Zellen, zum Beispiel von Fibroblasten, nicht kontrolliert werden. Hinzu kommt der limitierende Faktor der Zellausbeute, um das System an zahlreichen Kombinationen zu testen (Weisenthal und Kern, 1991; Pieters, 1990; Volm, 1988).

Ein vielversprechendes Konzept zur Analyse von Tumorzellen und ihrer Sensitivität gegen ausgewählte Cytostatika bietet sich über die bioluminometrische Messung des ATP-Gehalts, bei der direkt der Energieladungszustand der Zellen bestimmt wird (Andreotti et al., 1991; Bellamy, 1992; Hunter et al., 1993). Der ATP-Gehalt der Zelle sinkt mit dem Eintritt in einen statischen Zustand erheblich und ist sehr sensitiv nachweisbar (Sevin, 1993).

Die luminometrische ATP-Bestimmung zum Nachweis mitochondrialer Zellaktivität erfolgt üblicherweise nach der Isolierung und Präparation des aus einem operativen Eingriff stammenden Tumorgewebes, welches mechanisch zerkleinert und folgend enzymatisch in einer Tumordissoziierungslösung dissoziiert wird. Durch diesen Vorgang werden kleine Zellaggregate und Einzelzellen hergestellt. Der gesamte Vorgang nimmt in den üblichen Tests eine Zeitdauer von 4 bis 18 Stunden ein; durch die lange Inkubation entsteht eine große Menge an Zelldebris. Die eigentliche luminometrische ATP-Bestimmung erfolgt nach dem Aufschluß der dissoziierten Zellen. Die gemessenen Werte geben einen Hinweis auf die Zellaktivität der untersuchten Tumorprobe.

Bei luminometrischen ATP-Bestimmungen von Tumorgeweben kann also zum Beispiel prätherapeutisch ein Cytostatikum zugesetzt werden, um dessen Wirksamkeit auf das zu untersuchende Tumorgewebe zu bestimmen. Hierbei werden die 4 bis 18 Stunden in der Tumordissoziierungslösung verweilenden Zellen in eine Kultivierungslösung gegeben, die ein Cytostatikum enthält und anschließend deren Zellaktivität mittels luminometrischer ATP-Bestimmung gemessen. Bei den kommerziell angebotenen Assays erfolgt die ATP-Bestimmung durch die Messung an einem Luminometer in Einzelröhrchen, wobei die Überstände der Zellen in den Kulturgefäßen vorher abpipettiert werden. Hier ist somit die Seitenstrahlung der Lumineszenz auf die Eigenlumineszenz der verwendeten Röhrchen reduziert, es ist aber ein großer Pipettieraufwand erforderlich (in einer Microtiterplatte 96 mal), wodurch die Fehlerquote erheblich ansteigt. Die Auswertung von kommerziell erhältlichen Assays zeigt insbesondere bei der Zugabe von einem oder mehreren Cytostatika hohe Varianzen, diese haben einen störenden Einfluß auf die Interpretation der Ergebnisse.

So beschreibt Andreotti et al. (1995) ein Verfahren zum Testen der Chemosensitivität von humanen Tumoren in Microtiterplatten über die Bestimmung mittels eines ATP-Lumineszenzassays. Weitere Verfahren werden in Kurbacher et al. (1996 a), Cree et al. (1996), Kurbacher et al. (1996 b) und in Satori (1997) dargelegt. All diesen beschriebenen Tests ist gemeinsam, daß sie nicht auf alle Zelltypen anwendbar sind. Sichere Ergebnisse liefern die Tests vor allem bei Entartungen der Zellen des weiblichen Urogenitaltraktes und bei Brustgewebszellen. Seltene Tumorerkrankungen, wie das Unterzungengrund-Carcinom sind den bisherigen Tests überhaupt nicht zugänglich. Aber auch eine der schwersten Krebserkrankungen, mit einer 5-Jahres-Überlebensrate von unter 10 % der Leberkrebs, ist mit kommerziellen Tests nur schwer zu bestimmen. Die Kultivierung von entarteten Leberzellen gelingt nicht in solch einem Maße, daß eine in-vitro Cytostatikatestung an ihnen mit Erfolg vorgenommen werden könnte. Bei den Tumorerkrankungen der großen Organe, wie zum Beispiel dem Leberkrebs, ist ein weiteres Problem darin zu sehen, daß die Leberkrebszellen nur isoliert betrachtet werden. Allen beschriebenen Tests ist des weiteren gemeinsam, daß die Kultivierung der zu testenden Zellen über einen längeren Zeitraum (ca. 4 Tage) nicht gelingt, da die Zellen in ihrer Vitalität durch die lange Dissoziierungszeit bereits stark beeinträchtigt sind. Diese Tests können nur zum Messen einer ATP-Aktivität von potentiellen und/oder echten Tumorzellen verwandt werden, jedoch nicht zum Bestimmen der Veränderung der ATP-Aktivität über einen längeren Zeitraum in Gegenwart eingesetzter Cytostatika. Insbesondere für Osteosarcome, Pancreascarcinome, Leiomyosarcome und Histiocytome fehlen verläßliche Bestimmungsmethoden. Seltene Weichteilsarcome (zum Beispiel das Rhabdomyosarcom) können mit den bisherigen Methoden schwer, das seltene Unterzungengrund-Carcinom überhaupt nicht bestimmt werden.

Es ist zudem mit den bekannten Tests nicht möglich, mit geringen Materialmengen aus sogenannten minimalinvasiven Verfahren, wie sie zum Beispiel bei der Biopsie gewonnen werden, sichere Aktivitätsbestimmungen durchzuführen. Aber auch Ergußmaterial aus Asciten- oder Pleuralpunktaten war bisher reproduzierbaren Tests zur ATP-Bestimmung nicht verläßlich zugänglich.

Das der vorliegenden Erfindung zugrunde liegende technische Problem besteht also darin, ein insbesondere prätherapeutisch einsetzbares und gut reproduzierbares Verfahren zum Bestimmen der Aktivität mindestens eines Cytostatikums gegenüber Tumormaterial bereitzustellen, das eine individuelle patientenbezogene Bestimmung der Wirksamkeit eines Cytostatikums oder einer Kombination verschiedener Cytostatika, eine verkürzte Präparationsprozedur und eine Optimierung der Zellisolierung- und Vereinzelung ermöglicht sowie durch diese verkürzte Präparationszeit die Vitalität der Zellen erhöht und so eine lange Kultivierungszeit der zu testenden Zellen in Gegenwart mindestens eines Cytostatikums gestattet; außerdem soll der Test für möglichst viele unterschiedliche Zelltypen in geringer Menge auch in Gegenwart nicht-entarteter Zellen, in sogenannten heterogenen Kulturen, so anwendbar sein, daß die ATP-Bestimmung direkt an der Kulturplatte ohne Pipettierzwischenschritte erfolgen kann.

Die vorliegende Erfindung löst dieses technische Problem durch die Bereitstellung eines Verfahrens zum Bestimmen der Aktivität mindestens eines Cytostatikums gegenüber isoliertem Tumormaterial in Form von Tumorgewebe oder malignem Ergussmaterial, wobei das Verfahren die folgenden Schritte umfaßt:
a) das Zentrifugieren des Ergussmaterials zur Anreicherung der darin enthaltenen Zellen oder das mechanische Zerkleinern des Tumormaterials,
b) das enzymatische Dissoziieren des Tumormaterials, wobei das Tumormaterial in einer Dissoziierungslösung enthaltend 500000 bis 730000 units/100 ml Collagenase, 3500 bis 6500 units/100 ml DNAse und 0,2 bis 1,6 Gew.-% BSA sowie gegebenenfalls eine Protease, Carbohydrase und/oder Lipase über einen Zeitraum von 10 bis 120 Minuten inkubiert wird, der ausreicht, eine Suspension von Einzelzellen herzustellen,
c) die Zugabe des zu bestimmenden mindestens einen Cytostatikums zu der erhaltenen Suspension,
d) das Kultivieren der Suspension in einem Kulturmedium in Gegenwart des Cytostatikums,
e) das Aufschließen der kultivierten Einzelzellen durch Tumorextraktionslösung unter Zugabe eines Lumineszenz- und/oder eines photosensibilisierten Farbstoffes und
f) das Messen der ATP-Aktivität anhand der auftretenden Lumineszenz.

Die erfindungsgemäß ermöglichte Gewinnung einer reinen Einzelzellsuspension gegenüber der mit bisherigen Dissoziierungsmitteln herstellbaren Zellagglomerat/Einzelzellsuspension mit hohem Anteil an Zelldebris gewährleistet einen bedeutenden analytischen Vorteil, da die Bestimmung der Zellzahl dadurch einen höheren Grad an Exaktheit erreicht und die Zellaussaat in Kultivierungsplatten besser reproduzierbare Ergebnisse liefert, so daß die folgende Bestimmung des ATP-Gehaltes verläßlicher wird.

Das erfindungsgemäße Verfahren zeichnet sich durch eine hohe Sensitivität aus, die aufgrund der verwendeten bioluminometrischen ATP-Bestimmung in ausgezeichneter Weise mitochondriale Zellaktivität nachweist. Das erfindungsgemäße Verfahren zeichnet sich darüber hinaus durch eine gute Reproduzierbarkeit aus, welche unter anderem auf einer Optimierung der Gewebepräparationsart unter Einschluß eines enzymatischen Verdaus beruht, der innerhalb kürzester Zeit die Gewebeproben zu Einzelzellsuspensionen dissoziiert. Dadurch wird die Vitalität der Zellen erhalten, was durch ein langes Inkubieren mit aktiven Enzymen nicht möglich ist. Erfindungsgemäß werden Zellkonglomerate vermieden, so daß diese eine genaue Zellzahlbestimmung nicht stören und auch einer Zellaussaat nicht im Wege stehen, wodurch andernfalls bei einer späteren quantitativen ATP-Bestimmung Meßfehler auftreten könnten. Das erfindungsgemäße Verfahren eignet sich also für eine prätherapeutische Cytostatikum-Testung auf zweidimensionaler Kulturbasis für Einzelpräparate und Kombinationstherapien an Kliniken und onkologischen Praxen und durch behandelnde Ärzte. Selbstverständlich ist es auch möglich, das erfindungsgemäße Verfahren für die Cytostatika-Testung für den Einsatz in der Hyperthermie-Therapie einzusetzen sowie gegebenenfalls so zu erweitern, daß in-situ-Bedingungen soweit wie möglich imitiert werden können, beispielsweise durch Kokultivierung mit Zellen unterschiedlicher Gewebe beispielsweise Leberzellen in Form einer Leberpassage in-vitro. Das erfindungsgemäße Verfahren ermöglicht darüber hinaus eine zeitlich erheblich verkürzte und insbesondere finanziell attraktive Bestimmung von ATP in Tumormaterialien in Individuen-spezifischer Weise. Schließlich ermöglicht die erfindungsgemäße Verfahrensweise die verläßliche ATP-Bestimmung in allen Zelltypen, insbesondere entarteten Zellen.

Im Zusammenhang mit der vorliegenden Erfindung ist unter Tumormaterial jeder Zellverband oder jede Ansammlung freier Zellen menschlichen oder tierischen Ursprungs zu verstehen, der aber die mindestens eine Zelle aufweist, die in irgendeiner Form entartet ist. Das heißt, aufgrund physikalischer, biologischer und/oder chemischer Einflüsse, keine normale Entwicklung und/oder kein geregeltes Wachstum aufweist. Selbstverständlich kann das zu untersuchende Material auch aus einer Zellkultur stammen.

Im Zusammenhang mit der vorliegenden Erfindung wird unter Lumineszenz jede emittierte elektromagnetische Strahlung verstanden, die auftritt, wenn der zum luminometrischen Messen verwandte Stoff mit höherfrequenter Strahlung angeregt wird; Fluoreszenz und Phosphoreszenz sind gleichermaßen eingeschlossen.

Das erfindungsgemäße Verfahren geht vorn isoliertem Tumormaterial, also Tumorgewebe oder freie Zellen, insbesondere malignes Ergussmaterial, aus. Im Falle von Tumorgewebe kann dies Material beispielsweise durch Herausschneiden, Biopsie oder sonstige übliche Entnahmeverfahren erhalten worden sein. Im Falle von in Körperflüssigkeiten vorliegenden freien Zellen kann dies Material durch Absaugen, Biopsie oder sonstige übliche Entnahmeverfahren erhalten worden sein, wobei anschließend vorzugsweise ein Abzentrifugieren, Filtrieren oder sonstiges Trennen der Flüssigkeit von den in der Flüssigkeit vorhandenen freien Zellen durchgeführt wurde, so daß für den darauf folgenden Schritt weitgehend von Flüssigkeit befreites Zellmaterial zur Verfügung steht. Es kann sich jedoch auch um Material handeln, bei dem der Verdacht besteht, daß es sich um krankhaftes Gewebe und/oder entartete Zellen handelt.

Die vorliegende Erfindung geht von isoliertem Material aus, wobei unter dem Begriff Isolieren also einerseits die Entnahme aus einem menschlichen oder tierischen Körper oder einer Zellkultur sowie andererseits gegebenenfalls auch das Abtrennen von Flüssigkeit von der entnommenen Probe verstanden wird. Vorhandenes Gewebe wird mechanisch zerkleinert, um so die Gewebeoberfläche zu vergrößern. Durch die Oberflächenvergrößerung des Gewebes ist dieses weiteren Behandlungsschritten leichter zugänglich. Dies bezieht sich insbesondere auf Verfahrensschritte, die dazu dienen, einzelne Zellen aus dem Tumorgewebe herauszulösen, mit dem Ziel, letztendlich keinen Zellverband mehr vorliegen zu haben sondern nur noch Einzelzellen. Die Gewinnung von Einzelzellen in einer Lösung, der sogenannten Einzelzellsuspension, wird nach dem mechanischen Zerkleinern vor allem durch eine enzymatische Dissoziierung des mechanisch aufbereiteten Tumorgewebes in der erfindungsgemäßen Dissoziierungslösung erreicht. Die Dissoziierung erfolgt unter Verwendung einer speziellen wäßrigen Dissoziierungslösung enthaltend 500000 bis 730000 units/100 ml Collagenase, insbesondere 570000 units/100 ml Collagenase, 0,2 bis 1,6 Gew.-% BSA, insbesondere 0,4 Gew.-% BSA und 3500 bis 6500 units/100 ml DNAse, insbesondere 5000 units/100 ml DNAse (ml-Angaben und Gew.-% bezogen auf die gesamte wäßrige Lösung, insbesondere Puffer D). Die Inkubation dauert so lange, bis die vormals im Zellverband vorliegenden Tumorgewebszellen vollständig als Einzelzellen vorliegen. Hierzu werden alle 15 Minuten Aliquots entnommen und untersucht. Die gewonnenen Einzelzellen werden in Gegenwart mindestens eines Cytostatikums kultiviert. Zeitdauer und Kultivierungsparameter werden tumor- und patientenspezifisch variiert. Das Aufschließen der Einzelzellen erfolgt mit einer Tumorextraktionslösung unter zeitgleicher oder kurz zeitversetzter Zugabe eines Lumineszenz- und/oder eines photosensibilisierten Farbstoffes. Bestimmte Kompartimente oder Substanzen der aufgeschlossenen, Einzelzellen binden die Lumineszenz- und/oder photosensibilisierten Farbstoffe. Durch Bestrahlen dieser Farbstoffe mit höherfrequenter elektromagnetischer Strahlung erfolgt eine Emission von Lumineszenz. Diese auftretende Lumineszenz wird gemessen und gibt so einen Hinweis auf die gebundenen Farbstoffe und somit auf die ATP-Aktivität der aufgeschlossenen Einzelzellen.

Die Messung der ATP-Aktivität anhand der auftretenden Lumineszenz gibt also ein Signal, welches Rückschlüsse auf die Anzahl der angewachsenen und kultivierten Einzelzellen gibt. Da dies direkt abhängig von dem verwandten Cytostatikum ist, kann anhand des gemessenen Lumineszenzsignals ein Rückschluß auf die Wirksamkeit des Cytostatikums auf das Tumorgewebe erfolgen.

Die Erfindung betrifft demgemäß ein Verfahren, bei dem das Tumormaterial entweder aus Tumorgewebe und/oder freie Zellen besteht, wobei die freien Zellen insbesondere malignes Ergußmaterial sind. Im Sinne der Erfindung ist Tumorgewebe, jedes von Epidermalgewebe, Muskelgewebe, Nervengewebe und/oder dem Stütz- und/oder Bindegewebe abstammendes Gewebe. Gewebe zeichnen sich hierbei durch annähernd gleichartig differenzierte Zellen aus, die zusammengeschlossen sind. Jedes dieser Gewebe, was mindestens eine entartete Zelle aufweist, ist im Sinne der Erfindung Tumorgewebe. Eine Zelle ist unter anderem dann entartet, wenn sie veränderte Stoffwechselprozesse aufweist; das heißt, die Zelle gewinnt beispielsweise ab einem bestimmten Zeitpunkt durch biologische, chemische und/oder physikalische Einflußnahme ihre Energie auch aus Gärungsprozessen. Eine Zelle kann auch dann als entartet gelten, wenn sie ein modifiziertes immunologisches Verhalten zeigt; dies können zum Beispiel Veränderungen der Zelloberflächenstrukturen sein. Aber auch jede Änderung der Expressionsaktivität der Zelle ist im Sinne der Erfindung ein Indikator für eine Veränderung des geregelten Wachstums und der normalen Entwicklung der Zelle und somit ein ausreichender Grund, die Zelle als Krebszelle oder als entartete Zelle aufzufassen; eine verstärkte Expression von Onkogenen führt ebenso zur Entartung der Zelle wie eine verminderte Expression von Suppressorgenen. Im Sinne der Erfindung kann Tumormaterial auch aus sogenannten freien Zellen bestehen, diese können amöboide Wanderzellen, alle Zellen des Blutes und der Blutgerinnung wie auch omnipotente und pluripotente Zellen sein, aber auch alle Zellen, die respiratorische Pigmente aufweisen. Als freie Zellen im Sinne der Erfindung sind auch alle Zellen des Immunsystemes aufzufassen, die nicht als Gewebe vorliegen, wie zum Beispiel die aus dem Knochenmark stammenden B- und T-Lymphocyten. Alle entarteten Zellen, die nicht gewebeartig vorliegen und in Flüssigkeitsansammlungen vorhanden sind, gelten im Sinne der Erfindung als malignes Ergußmaterial. Hierzu ist es unwesentlich, ob die Flüssigkeitsansammlung unmittelbar oder nur mittelbar durch einen Tumor verursacht wird. Ebenso ist es im Sinne der Erfindung unwesentlich, ob das zu untersuchende Material gutoder bösartig ist, auch jedes gutartige Geschwulstmaterial ist im Sinne der Erfindung Tumormaterial.

Das erfindungsgemäße Verfahren betrifft in einer besonderen Ausführungsform ein Verfahren, um Tumormaterial aus minimal- und microinvasiven Eingriffen zu untersuchen. Minimal- und microinvasive Eingriffe sind alle Eingriffe, die dazu dienen, Gewebe und/oder Zellen zu gewinnen, welche Entwicklungsund Wachstumsbesonderheiten aufweisen. Auch sämtliches gewonnenes Flüssigkeitsmaterial, welches den Körper mit oder ohne weiteres ärztliches Zutun verläßt, gilt im Sinne der Erfindung als minimalund/oder microinvasiv gewonnenes Material, hierbei kann es sich um Zellen handeln, die Körperöffnungen passieren; wobei Körperöffnungen Wunden, wie auch künstliche oder natürliche Öffnungen sein können. Jedoch auch jedes Gewebe oder alle freien Zellen einschließlich Zellaggregate, die durch chirugische Eingriffe gewonnen werden, um entweder einen Teil oder das gesamte Tumormaterial zu entfernen, gelten im Sinne der Erfindung als minimal- und microinvasive Eingriffe.

In einer weiteren Ausführungsform betrifft die Erfindung ein Verfahren zum Bestimmen der Aktivität mindestens eines Cytostatikums gegenüber Gewebe aus minimalinvasiven Eingriffen, welches Biopsiematerial, Pleural- und/oder Ascitespunktatmaterial ist. Diese Tumormaterialien weisen weniger Zellen auf, als Material, welches durch konventionelle operative Eingriffe gewonnen wird.

Die Erfindung betrifft demgemäß ein Verfahren zur Bestimmung mindestens eines Cytostatikums gegenüber Tumormaterial, wobei die Tumorzellen in Gegenwart eines Cytostatikums kultiviert werden. Tumorzellen haben besondere physiologische Anforderungen an die Kultivierung. Jede Beeinträchtigung ihrer Vitalität erlaubt keine optimale Kultivierung mehr, insbesondere dann, wenn die Kultivierung zusammen mit dem Cytostatikum erfolgt. Deshalb ist es erforderlich, den entarteten Zellverband so zu dissoziieren, daß eine vollständige Vereinzelung der Zellen in kurzer Zeit realisiert wird. Dies wird erfindungsgemäß durch eine Inkubation mit einer Dissoziierungslösung ermöglicht, die Collagenase, BSA und DNAse umfaßt.

Die Erfindung betrifft ein Verfahren, wobei die Dissoziierungslösung gegebenenfalls eine Protease, Carbohydrase und/oder Lipase umfaßt. Für die Bestimmung der ATP-Aktivität ist eine vollständige Vereinzelung der Zellen erforderlich, die erst eine genaue Bestimmung der Zellzahl wie auch eine optimale Kultivierung ermöglicht. Da Tumorzellen zusammen mit sehr kompaktem Gewebe auftreten können, ist es zwingend, daß die Dissoziierungslösungen, alle Verbindungen zwischen biologischen Bausteinen aufzulösen vermag; bei den biologischen Bausteinen kann es sich um Proteine, Kohlenhydrate und/oder Lipide handeln. Die Dissoziierungslösung kann deshalb so zusammengesetzt sein, daß sie entweder nur Proteine oder Kohlenhydrate oder Lipide zu dissoziieren vermag beziehungsweise mehrere oder alle dieser Bausteine.

In einer besonderen Ausführungsform kann vorgesehen sein, daß die Protease Pronase E, die Carbohydrase Hyaluronidase und die Lipase Pankreatin ist. Die Dissoziierungslösung kann Pronase E, Hyaluronidase oder Pankreatin, beziehungsweise einzelne Komponenten oder auch alle Bestandteile umfassen. Je nach Tumor kann die Dissoziierungslösung also Pronase E und/oder Hyaluronidase und/oder Pankreatin umfassen. Alle Enzyme können jedoch erfindungsgemäß auch wegen anderer Aktivitäten eingesetzt werden; so besitzt zum Beispiel Pankreatin auch eine Amylase-, Trypsin-, Ribonuclease- und Protease-Aktivität und Hyaluronidase kann zahlreiche andere polysaccharidspaltenden Enzymaktivitäten aufweisen. Erfindungsgemäß ist vorgesehen, daß die Dissoziierungslösung Pankreatin in einer Menge von 0,03 bis 0,07 Ges.-%, Pronase E in einer Menge von 150 bis 345 units/100 ml und/oder Hyaluronidase in einer Menge von 300 000 bis 520 000 units/100 ml enthält.

Erfindungsgemäß beträgt die Zeit, die benötigt wird, um das Tumormaterial enzymatisch zu dissoziieren, 10 bis 120 Minuten. Diese auch als Inkubationszeit bezeichnete Zeitspanne ist so gewählt, daß sehr wenig Zelldebris entsteht und somit das Meßergebnis kaum oder nicht verfälscht werden kann, da so der Farbstoff nicht unspezifisch an "Zelltrümmer" und ihre Komparimente und Inhaltsstoffe binden kann. Wenn eine Kultivierung der Tumorzellen angestrebt wird, um Cytostatika-Testungen durchzuführen, können die so enzymatisch dissoziierten Tumorzellen mit Erfolg verwandt werden, da ihre Vitalität durch die 10 bis 120 minütige Inkubation in der Dissoziierungslösung nur unwesentlich beeinflußt ist. Die Kultivierung der vitalen Zellen ist dadurch so optimiert, daß ein mögliches vermindertes Wachstum der Zellen nur auf die Wirkung des Cytostatikums zurückzuführen ist und nicht auf Defekte, die durch die Dissoziierungslösung initiiert wurden. Außerdem ist der Zeitraum so gewählt, daß er beispielsweise in einem klinischen Labor innerhalb eines Tages durchgeführt werden kann. Eine Übernacht-Inkubation in Abwesenheit von Personen, wodurch Fehlmessungen begünstigen werden könnten, ist somit nicht notwendig. Im klinischen Alltag stellt das einen großen Vorteil dar. Auch kann der gesamte kurze Ablauf des Verfahrens im Gegensatz zu den bisherigen Methoden von ein und derselben Person durchgeführt werden, so daß der subjektive Fehler der Messung immer gleich ist.

In einer weiteren bevorzugten Ausführungsform kann vorgesehen sein, daß die Kultivierung der Einzelzellen in einem Zeitraum von 3 bis 7 Tagen durchgeführt wird. Durch die kurze Zeitdauer der Dissoziierung sind die Zellen vital genug, um sehr lange kultiviert werden zu können. Da die Einzelzellen in Gegenwart des Cytostatikums kultiviert werden, kann auf eine vorgeschaltete Kulturphase verzichtet werden, die zu einer Konfluenz beziehungsweise Subkonfluenz führt, die wiederum die ATP-Bestimmung verfälscht, da der ATP-Gehalt in den Zellen durch Kontaktinhibition sinkt. Durch die Kultivierung der Einzelzellen über den Zeitraum von 3 bis 7 Tagen ist es möglich, eine gute Annäherung an die Dauer des Cytostatikums in den potentiellen Patienten zu erreichen. In Therapien verbleibt oder zirkuliert das Cytostatikum zirka eine Woche im Patienten beziehungsweise speziell im jeweiligen Tumor, in welches es appliziert wurde. Dadurch, daß die Testdauer in-vitro der Situation in-vivo angepaßt wurde, ist eine gute Einschätzung der biologischen Wirksamkeit des Cytostatikums gegenüber dem Tumormaterial des Patienten möglich. So kann für jeden einzelnen Patienten prätherapeutisch die Wirkung der Cytostatika in-vitro getestet werden.

In einer bevorzugten Ausführungsform kann vorgesehen sein, die gewonnenen Einzelzellen in ein konditioniertes Medium zu geben. Die Kultivierung solch spezieller Zellen wie der Tumorzellen verlangt besondere Kulturbedingungen. Neben der Modifikation der Oberfläche der Kulturgefäße ist die Veränderung der Lösung und des Mediums, in dem die Zellen kultiviert werden, eine besondere Form, um die Tumorzellen über die geforderte Zeitspanne optimal zu kultivieren.

In einer bevorzugten Ausführungsform wird das konditionierte Medium aus Kulturmedium hergestellt, indem nicht-entartete Zellen eingebracht werden. Diese geben Metaboliten und Mediatoren in das Kulturmedium ab. Die Tumorzellen, die in dieses Medium eingesät werden, sind dadurch physiologisch einer in-vivo-ähnlichen Situation angepaßt. Die mit diesen Tumorzellen durchgeführten Messungen geben somit eine an den Patienten angenäherte Situation wieder, vor allem wenn die Tumorzellen und die nicht-entarteten Zellen spezifisch aus einem Patienten stammen.

In einer weiteren bevorzugten Ausführungsform kann vorgesehen sein, daß die Kultivierung der Einzelzellen als Kokultivierung erfolgt. Viele Tumorzellen benötigen die Anwesenheit von nicht-tumorigenen Zellen, um sich zu etablieren und zu proliferieren. Durch die heterogene Kultur -die sogenannte Kokultur- wird zum einen die Anwachsrate der Tumorzellen verbessert und zum anderen wird das gesamte System dem physiologischen Status angenähert. Da die Wirkung der Cytostatika unter anderem durch die paracrine und autocrine Interaktion der unterschiedlichen Zelltypen erheblich beeinflußt wird, ist durch die Annäherung an dieses System eine bessere Testung der Cytostatika möglich.

Die Kokultivierung erfolgt in einer bevorzugten Ausführungsform durch die gleichzeitige Kultivierung der tumorigenen und nicht-tumorigenen Zellen. Da das Cytostatikum in-vivo auf gesunde wie auf Krebszellen wirkt und dadurch spezielle Mataboliten entstehen können, die das Wachstum und die Entwicklung der Krebszellen positiv oder negativ beeinflussen, ist die gleichzeitige Kokultivierung eine Möglichkeit, die Situation im Organismus wiederzuspiegeln und experimentell modellhaft zu erfassen.

Die Erfindung betrifft demnach auch ein Verfahren, indem die Kokultivierung von tumorigenen und nicht-tumorigenen Zellen mit oder ohne räumliche Trennung erfolgen kann. Bei der Kokultivierung kann vorgesehen sein, daß eine Vermischung der Zellen erfolgt oder daß diese verhindert wird. Ein Vermischen der unterschiedlichen Zelltypen kann zum Beispiel bei Tumorzellen sinnvoll sein, die gesunde Zellen direkt infiltrieren. Bei Tumorzellen, die für ihr Wachstum in-vivo eine Passage durch Organe benötigen; kann eine Kokultivierung _mit einem Vermischen der unterschiedlichen Zelltypen, ein Modell sein, um diese Situation in-vitro darzustellen.

In einer weiteren Ausführungsform kann vorgesehen sein, daß die Trennung der kokultivierten Zellen durch eine für Metaboliten durchlässige Membran erfolgt. Interzelluläre Regulationsmechanismen, insbesondere die über Metaboliten vermittelten, können so erfaßt und im System experimentell aufgenommen werden. Ein direkter Kontakt wie auch ein Durchmischen der Zellen ist ausgeschlossen. Wechselwirkungen, die auf direktem Kontakt der unterschiedlichen Zelltypen beruhen, werden durch die Membran unterbunden. Die Membran kann so gewählt sein, daß sie Substanzen nach qualitativen Merkmalen passieren läßt; sie kann aber auch eine durchlässige Barriere sein, die nach der Größe - also quantitative Merkmalen - trennt.

In einer ganz besonderen Ausführungsform kann vorgesehen sein, daß die kokultivierten normalen Zellen Hepatocyten -auch als Hepatocytenplättchen bezeichnet- und/oder Fibroblasten sind. Die gezielte Verwendung der Leberparenchymzellen und/oder der fibroblastenartigen Zellen in der Kokultur mit Tumorzellen hat gegenüber der Prüfung im lebenden Organismus den Vorteil der einheitlichen chemischen und physikalischen Verhältnisse, der exakten Dosierung, der definierten Cytostatikakonzentration an der Targetzelle, der genauen Kontrolle der Expositionsdauer an der Zelle sowie der direkten Bestimmung der biologischen Effekte des Cytostatikums an der Tumorzelle mittels Mikroskopie und biochemischer Methoden. Die bei der Prüfung am lebenden Organismus besonders störenden Einflüsse durch Blutkreislauf, Hormonstatus und Nervensystem können vollständig ausgeschlossen werden. Durch die Kokultivierung von Hepatocyten und Tumorzellen kann insbesondere eine in-vitro-Leberpassage im Modell untersucht werden. Es kann sich als besonders geeignet erweisen, die Cytostatika direkt mit den Zellen in das Kulturmedium einzubringen. Auf eine vorgeschaltete Kulturphase der Zellen ohne Cytostatika wird verzichtet, da eine auftretende Kontaktinhibition der konfluenten Zellen die ATP-Produktion senkt. Die Veränderungen des ATP-Gehaltes der Zellen wären dann nicht mehr ursächlich auf das Cytostatikum zurückzuführen.

Die zu der luminometrischen ATP-Bestimmung eingesetzten Microplatten weisen eine 20% bis 90% geringere Eigenfluoreszenz gegenüber den herkömmlichen Microplatten auf. Dadurch daß diese Platten weniger elektromagnetische Strahlung emittieren, wenn sie durch Einstrahlung höherfrequenter elektromagnetischer Strahlung angeregt werden, beeinflussen sie die luminometrische ATP-Bestimmung nur unwesentlich. Jedes gemessene Lumineszenzsignal kommt somit durch das Auftreten angeregter Elektronenzustände während einer chemischen Reaktion am ATP der Mitochondrien der Tumorzellen zustande und nicht durch eine Reaktion an den verwandten Kulturplatten, jedes aufgenommene luminometrische Signal (zum Beispiel ein Emissionsspektrum) stammt von dem Lumineszenz- oder photosensibilisierten Farbstoff und nicht von der Eigenfluoreszenz der verwandten Materialien.

In einer bevorzugten Ausführungsform kann vorgesehen sein, daß die Microplatten mit Proteinen der extrazellulären Matrix beschichtet sind. Zur Verbesserung und Modifikaton der Oberfläche von Mikroplatten können diese mit diesen Proteinen vorbeschichtet werden. Bestimmte Beschichtungen machen die in-vitro-Kultivierung ausgewählter Tumorzellen erst möglich. Besonders bei tierischen Zellkulturen -den besonders adhärenten Zellen- spielt die Oberfläche des Kulturgefäßes eine große Rolle. Über die gesamte Tumorzelle sind unregelmäßig Ladungen verteilt; für das Anheften der Zellen ist deshalb die Ladungsdichte auf der Oberfläche der Kulturplatten wichtig. Für die Adhäsion der Tumorzellen sind mindestens zwei Ladungsträgertypen entscheidend, die bivalenten Kationen und/oder bestimmte Proteine. Physiologische Zellproteine -wie Proteine der extrazellulären Matrix- sind in-vivo entscheidend dafür verantwortlich, daß Zellen aneinanderhaften. Die extrazelluläre Matrix bildet den "Klebstoff", der die Zellen aber auch Strukturen wie Sehnen, Knorpel und Knochen zusammenhält. Aus diesem Grunde erhöht sich die Anwachsdichte der Tumorzellen in-vitro, wenn die Kulturgefäße mit extrazellulären Proteinen beschichtet werden. Die Kulturgefäße können außerdem einen ebenen Boden haben, damit sich die Zellen nicht in Vertiefungen ansammeln.

Die eingesetzten Microplatten weisen in einer besonders bevorzugten Ausführungsform der vorliegenden Erfindung eine Beschichtung mit Collagen und/oder Fibronectin auf. Hierdurch wird eine höhere Anwachsrate der Tumorzellen erzielt, wodurch Fehltestungen minimiert werden. Durch die Beschichtung mit Collagen und/oder Fibronectin werden außerdem unspezifische Bindungsstellen an der Oberfläche der Microplatten, insbesondere Microtiterplatten, "abgebunden". Somit ist es nicht möglich, daß der lumineszierende und/oder photosenibilisierte Farbstoff direkt an die Platte bindet und so das Meßergebnis beeinflußt. Eine Bindung des Farbstoffes kann deshalb nur an Kompartimente der aufgeschlossenen Tumorzellen erfolgen.

Erfindungsgemäß kann vorgesehen sein, daß der Lumineszenzfarbstoff der Chemolumineszenzfarbstoff Luciferin-Luciferase ist. Der Farbstoff Luciferin-Luciferase zeichnet sich durch eine sehr hohe Sensitivität aus.

Als ganz besonders vorteilhaft hat es sich erwiesein, daß das Verfahren in einem Zeitraum von 20 bis 500 Minuten, insbesondere im Zeitraum von 20 bis 240 Minuten, durchgeführt wird. Somit ist es möglich, die Präparationsprozedur innerhalb von sehr kurzer Zeit durchzuführen. Durch die kurze Bearbeitungszeit während des Dissoziierens des Tumormaterials wird die Vitalität der zu untersuchenden Zellen erhalten. Die vitalen Einzelzellen werden kultiviert, um den Einfluß der Cytostatika auf die kultivierten Tumorzellen zu untersuchen. Das gesamte Verfahren muß zeitlich so gestaltet sein, daß die in-vitro-Situation der in-vivo-Situation angepaßt ist.

In einer weiteren Ausführungsform betrifft die Erfindung ein Verfahren, bei dem die Kultivierung und/oder die Kokultivierung bei einer Temperatur erfolgt, bei der auch eine Hyperthermie-Therapie durchgeführt wird; wobei die Temperaturabfolge speziell auf einen bestimmten Patienten abgestellt ist. Bei der Hyperthermie-Therapie wird künstlich von außen die Körpertemperatur über die Normaltemperatur erhöht. Da Tumorzellen in der DNA-Synthesephase (Reduplikation) eine höhere Temperaturempfindlichkeit als normale Zellen aufweisen, kann diese Therapie genutzt werden, um durch selektive Erhitzung des Tumors oder als extrakorpörale Ganzkörperhyperthermie mit einer erzeugten Temperatur von über 41°C den Tumor abzutöten.

Dieses Abtöten des Tumors kann durch den Einsatz von Cytostatika beschleunigt und optimiert werden. Um das Cytostatikum patienten- und tumorspezifisch zu optimieren, muß prätherapeutisch analysiert werden, wie das Cytostatikum das Wachstum des Tumormaterials unter den bei der Hyperthermie-Therapie zu wählenden Temperaturen oder den speziell zu wählenden Temperaturabfolgen beeinflußt. Insbesondere erfolgt die prätherapeutische Analyse bei ansteigenden Temperaturen im Bereich von 37°C bis 41°C.

Die vorliegende Erfindung betrifft auch ein Verfahren zur luminometrischen ATP-Bestimmung von isoliertem potentiell entartete Zellen enthaltenden Material, insbesondere Tumormaterial. Das Verfahren umfaßt die folgenden Schritte:
a) gegebenenfalls das mechanische Zerkleinern des Tumormaterials, sofern es in Form von Gewebe vorliegt,
b) das enzymatische Dissoziieren des Tumormaterials, wobei das Tumormaterial in einer Dissoziierungslösung enthaltend 500000 bis 730000 units/100 ml Collagenase, 3500 bis 6500 units/100 ml DNAse und 0,2 bis 1,6 Gew.-% BSA über einen Zeitraum inkubiert wird, der ausreicht, eine Suspension von Einzelzellen herzustellen,
c) das Aufschließen der Einzelzellen durch Tumorextraktionslösung unter Zugabe eines Lumineszenz- und/oder eines photosensibilisierten Farbstoffes und
d) das Messen der ATP-Aktivität anhand der auftretenden Lumineszenz.

Das vorgenannte erfindungsgemäße Verfahren ermöglicht also durch das Bestimmen der mitochondrialen Aktivität von Zellen das Feststellen von Tumoreigenschaften eines zu untersuchenden potentiell entartete Zellen enthaltenden Materials.

In einer weiteren bevorzugten Weise kann vorgesehen sein, die Einzelzellsuspension vor dem Aufschließen der Einzelzellen immunomagnetisch so aufzureinigen, daß eine Anreicherung von entarteten Einzelzellen erreicht wird. Dadurch ist es möglich, daß nicht-entartete Zellen -wie zum Beispiel typische Zellen des stützenden Bindegewebes wie Fibroblasten- von der ATP-Bestimmung ausgeschlossen werden. Somit erfolgt die luminometrische ATP-Messung nur an den eigentlich entarteten Zellen. Der ATP-Wert der normalen Zellen fließt nicht in das Ergebnis der luminometrischen ATP-Bestimmung ein. Somit ergibt sich eine direkte Korrelation zwischen dem luminometrisch bestimmten ATP-Wert und der mitochondrialen Zellaktivität der Tumorzellen.

Von großer Bedeutung, um die Inkubationszeit möglichst kurz zu halten, ist die erfindungsgemäße Dissoziierungslösung, die sowohl für das Verfahren zur Bestimmung der Wirksamkeit eines Cytostatikums als auch zur ATP-Bestimmung von Tumormaterial eingesetzt wird. Diese umfaßt Collagenase, BSA und DNAse. Collagenase wird in einer Menge von 500000 bis 730000 units/100 ml, insbesondere 570000 units/100 ml, BSA in einer Menge von 0,2 bis 1,6 Gew.-%, insbesondere 0,4 Gew.-% und DNAse in einer Menge von 3500 bis 6500 units/100 ml, insbesondere 5000 units/ml eingesetzt (ml-Angaben und Gew.-% bezogen auf die gesamte wäßrige Lösung, insbesondere Puffer D).

Die vorliegende Erfindung betrifft außerdem eine Dissoziierungslösung, die eine Protease, Carbohydrase und/oder eine Lipase umfaßt, wobei die Protease Pronase E, die Carbohydrase Hyaluronidase und die Lipase Pankreatin ist. Pronase E wird in einer Menge von 150 bis 345 units/100 ml, insbesondere 237,5 units/100 ml, Hyaluronidase in einer Menge von 300000 bis 520000 units/100 ml, insbesondere 440000 units/100 ml und/oder Pankreatin in einer Menge von 0,03 bis 0,07 Gew.-%, insbesondere 0,05 Gew.-% eingesetzt (ml-Angaben und Gew.-% bezogen auf die gesamte wäßrige Lösung, insbesondere Puffer D).

Weitere vorteilhafte Ausgestaltungen ergeben sich aus den Unteransprüchen.

Die folgenden Beispiele und die dazugehörigen Figuren erläutern die Erfindung:

Die Figuren zeigen:
- Figur 1: ein Zwei-Kammer-Kokultivierungssystem mit Hepatocytenplättchen und Carcinomzellen in Kokultur und
- Figur 2: ein Zwei-Kammer-System für die Herstellung von konditioniertem Medium durch Hepatocytenplättchen.

### Beispiel 1

### 1. Ausgangsmaterial

Ausgangsmaterial ist isoliertes Tumormaterial, welches in ein Transportmedium eingebracht wird, um es von dem Ort der Entnahme zum Ort der Untersuchung zu transportieren. Tumormaterial aus Ergüssen mit einem hohen Flüssigkeitsanteil kann auch in.einem Transfusionsbeutel ohne Transportmedium gelagert werden.

### 2. Zusammensetzung des Tansportmediums:

Roswell-Park-Memorial-Institute (RPMI)-Medium
10 % Fetales Kälberserum (FCS)
1 % Gentamycin
1 % L-Glutamin

25 ml dieses Transportmediums liegen in einem Probenröhrchen (50 ml Volumen) der Firma Falcon vor. In diese Falcon-Röhrchen wird das entsprechende Tumormaterial für die Testung gegeben. Bei Pleuralpunktat/Ascites-Ergulsmaterial erfolgt der Transport wie erwähnt in einem Transfusionsbeutel.

Das so gelieferte Tumormaterial wird, so es als Gewebe vorliegt, im folgenden in sterilen Greiner-Petrischalen (10 cm Durchmesser) mit autoklaviertem Präparierbesteck (Skalpell, Pinzetten) mechanisch aufbereitet. Hierbei wird das Tumormaterial mechanisch zerkleinert, um die Oberfläche des Tumormaterials für die folgenden Verarbeitungsschritte zu vergrößern. Bei Pleuralpunktaten- und Ascitesmaterial ist diese mechanische Tumoraufbereitung nicht erforderlich, da es sich hierbei um Ergußmaterial aus dem Patienten handelt. Pleuralpunktate und Ascitesmaterial werden statt dessen zentrifugiert, um die in diesem Material enthaltene freien Zellen anzureichern.

### 3. Patienten- und tumorspezifische Dissoziierung des Tumormaterials

Je nach Probe wird entschieden, ob eine Ein-Schritt oder Zwei-Schritt-Dissoziierung des Gewebes erfolgt. Bei der Zwei-Schritt-Dissoziierung wird nach dem ersten Schritt der enzymatischen Dissoziierung eine weitere separate Inkubation mit einer Dissoziierungslösung (insbesondere Dissoziierungslösung E, siehe unten) für 1 bis 20 Minuten durchgeführt.

### Dissoziierungslösungen:

Alle Dissoziierungslösungen werden in einem Puffer D angesetzt.

| Zusammensetzung von Puffer D: | |
|---|---|
| NaCl | 8000 mg |
| KCl | 373 mg |
| Na₂HPO₄x2H₂O | 125 mg |
| Hepes | 5950 mg |
| D-Glucose | 2000 mg |
| gelöst in Aqua dest., pH 7,35 | 1000 ml |

| Dissoziierungslösung A: | |
|---|---|
| Collagenase CLSII, "Worthington Type" | 50 mg |
| BSA | 80 mg |
| DNAse | 0,5 mg |
| gelöst in Puffer D | 20 ml |

| Dissoziierungslösung B: | |
|---|---|
| Collagenase CLSII, "Worthington Type" | 50 mg |
| BSA | 80 mg |
| Hyaluronidase | 20 mg |
| DNAse | 0,5 mg |
| gelöst in Puffer D | 20 ml |

| Dissoziierungslösung C: | |
|---|---|
| Collagenase CLSII, "Worthington Type" | 50 mg |
| BSA | 80 mg |
| Pronase E | 0,5 mg |
| DNAse | 0,5 mg |
| gelöst in Puffer D | 20 ml |

| Dissoziierungslösung D: | |
|---|---|
| Collagenase CLSII, "Worthington Type" | 50 mg |
| BSA | 80 mg |
| Pronase E | 0,5 mg |
| Hyaluronidase | 20 mg |
| DNAse | 0,5 mg |
| gelöst in Puffer D | 20 ml |

| Dissoziierungslösung E: | |
|---|---|
| Pankreatin4xNF | 2 ml |
| gelöst in Puffer D | 18 ml |

Wie bereits ausgeführt, wird Dissoziierungslösung E im Zwei-Schritt-Verfahren als zweite Lösung eingesetzt.

Die Inkubationszeit der Dissoziierung mit einer der dargestellten Dissoziierungslösungen beträgt 10 bis 120 Minuten; sie ist stark patienten- und tumorspezifisch. Während der Inkubation werden alle 10 bis 15 Minuten Aliquots entnommen, die mikroskopisch auf den Grad der Vereinzelung -also dem Grad der Dissoziierung- beurteilt werden. Die Inkubation wird beendet, wenn das mikroskopische Bild der untersuchten Tumorprobe Material zeigt, welches vollständig dissoziiert ist. Die enzymatische Dissoziierung erfolgt in Glasgefäßen aus hitzebeständigem Glas in unterschiedlichen Zeitrahmen, die dem jeweiligen Patienten und/oder Tumor angepaßt sind.

Handelt es sich bei der Gewebe- oder Ergußprobe um ein Mamma-, Cervix-, Endometrium-, Ovarial-, Prostata-, Bronchial- und/oder Lungen-Carcinom, wird das Tumormaterial in einem Zeitraum von 15 bis 45 Minuten in Dissoziierungslösung A inkubiert.

Bei Rhabdomyosarcom, Leiomyosarcom und Histiocytomen wird in einem Zeitraum von 60 bis 90 Minuten patienten- und probenspezifisch in Dissoziierungslösung B inkubiert. Bei Leiomyosarcomen ist es für das Erreichen einer vollständigen Dissoziierung erforderlich, einen weiteren Inkubationsschritt über 15 Minuten in Dissoziierungslösung D folgen zu lassen.

Bei Pankreas-, Colon- und Sigma-Carcinomen erfolgt im Zeitraum von 20 bis 60 Minuten eine Inkubation in Dissoziierungslösung C.

Osteosarcome und Unterzungengrund-Carcinome werden in der Dissoziierungslösung D für 40 bis 90 Minuten inkubiert.

Für einige Sarcome und Carcinome ist eine weitere Dissoziierung in der Dissoziierungslösung E erforderlich. So werden Mamma-, Ovarial-, Pancreas- und Unterzungengrund-Carcinome noch einmal 2 bis 15 Minuten in Dissoziierungslösung E inkubiert, bis im Microskop eine Einzelzellsuspension wahrnehmbar ist.

### 4. Zellzählung und Bestimmung der Qualität der Zellen

Die mit verschiedenen Dissoziierungslösungen hergestellten Einzelzellsuspensionen werden im folgenden einheitlich weiterverarbeitet. Die gewonnenen Einzelzellen werden nach einer standardisierten Methodie am CASY-Coultercounter gezählt, die Daten zur Aussage der Qualität der Zellen werden EDVtechnisch mit einem Algorithmus erfaßt. Die Zellen werden je nach Zellausbeute und Menge der angeforderten Cytostatikatestungen in einer Konzentration von 1-2 x 10⁵ Zellen/100 µl pro Loch in 96-Loch-Kulturplatten oder Microplatten (Costar-Corning-Spezialplatten, flacher Boden, steril, weiß für Luminezenz) ausgesät. Zusätzlich werden zwei Kulturflaschen (175 ml) in der einmal im Test verwendeten Zellkonzentration (gekennzeichnet als Flasche -) und die gesamte Restmenge aufnehmend (gekennzeichnet als Flasche +) zur Sterilitätskontrolle und Anwachsprüfung angesetzt und für 4 Tage in einem Inkubator bei 37°C und 5 % CO₂ kultiviert.

Ausgehend von einer Standardtestung, bestehend aus zwei Kulturplatten mit insgesamt sechs möglichen Cytostatika in sieben verschiedenen Konzentrationen, einer Positivkontrolle, einer Negativkontrolle und pro Platte eine ATP-Standardkurve, kann der Test jeweils um vier weitere Cytostatika (= eine Platte) unbegrenzt erweitert werden. Die ATP-Standardkurve, die für jede Platte gemessen werden muß, wird in zwölf verschiedenen Konzentrationen erfaßt. Die Positivkontrolle wird dadurch bestimmt, daß nur Zellen mit dem Kulturmedium vorgegeben und gemessen werden; die Negativkontrolle erfolgt durch die Messung von den Zellen, denen ein ATP-Inhibitor zugesetzt wurde.

### 5. Messung

Nach 4 Tagen unter Kulturbedingungen (37°C, 5 % CO₂) wird das Kulturmedium mit dem Cytostatikum oder den Cytostatika abgenommen und die Zellen werden mit 25 µl Lysispuffer der Firma Boehringer Mannheim lysiert, wodurch das in den Zellen befindliche ATP freigesetzt wird. Im folgenden werden 150 µl Puffer D und 25 µl Luciferin-Luciferase-Reagenz (Firefly) der Firma Boehringer Mannheim zugegeben. In der so behandelten Platte wird in einem SpectroFluor Reader der Firma Tecan die vorhandene Lumineszenz direkt gemessen.

### 6. Auswertung

Die Auswertung erfolgt über einen mathematischen Algorithmus (MS Office 97 Professional Excel) anhand eigens erstellter Auswertungsformulare. Die ATP-Inhibition, die Wertung der Wirkung, die graphische Darstellung der ATP-Inhibition und die graphische Darstellung der Area Under Curve (AUC), werden tabellarisch aufgeführt und dem behandelnden Arzt zugesandt.

### Beispiel 2:

### Die zweidimensionale Primärkultur im Hyperthermie-Test

Dieses zweidimensionale Testsystem findet Anwendung im Bereich der Hyperthermie-Therapie. Um die Wirkung der Cytostatika unter den veränderten Temperaturbedingungen der Hypertheramie-Therapie zu testen, wird während der Testperiode ein Temperaturspektrum von 37°C bis 41°C abgedeckt. Hierzu ist es erforderlich, den Kulturansatz um den Faktor 5 zu erhöhen, das heißt, der Ansatz nach Beispiel 1 muß fünfmal geteilt werden, um ihn jeweils in einer separaten Probe den Temperaturen 37°C, 38°C, 39°C, 40°C und 41°C auszusetzen. Ein Unterschied zu Beispiel 1 besteht deshalb darin, daß die Zellausbeute geringer ist, da der gesamte Testansatz vor der Inkubation bei den unterschiedlichen Temperaturen fünfmal geteilt wird.

### Beispiel 3:

### Metaboliten-Test durch vorgeschaltete beziehungsweise integrierte in-vitro-Leberpassage

In dem Zwei-Kammer-System besteht die Möglichkeit, prätherapeutisch Cytostatika-Testungen durchzuführen. Eine Kokultivierungstechnik gestattet es, spezifisch die Metabolisierungsaktivität der eingesetzten Cytostatika zu untersuchen. In dem Zwei-Kammer-System besteht also die Möglichkeit, ein physiologisches System aufzubauen, in dem Tumorzellen durch die Anwesenheit von gesunden Zellen besser proliferieren können. So können in diesem Zwei-Kammer-System spezielle Fragestellungen zur interzellulären Regulationsmechanismen und der gegenseitigen Beeinflussung von Tumor- und Normal-Zellen über Metaboliten und/oder Mediatoren untersucht werden, da die interessierenden unterschiedlichen Zelltypen so kokultiviert werden können, daß entweder eine Vermischung oder aber keine Vermischung der einzelnen Zelltypen auftreten kann. Die Trennung der beiden kokultivierten Zelltypen wird eine Membran innerhalb des Kulturgefäßes erreicht.

Das physikalische Problem der geringen Adhärenz zum Objektträger liegt bei der Metaboliten-Testung nicht vor. Durch die Kokultivierung von embryonalen porcinen Hepatocytenplättchen -die sich im Kammereinsatz befinden- und Carcinomzellen -die auf dem Kammerboden kultiviert wurden- wird eine künstliche Leberpassage in-vitro entwickelt. Der Nachweis der zu erwartenden Metaboliten im Medium wird durch massenspektrometrische Analyse durchgeführt. Das in dieses Zwei-Kammer-System eingebrachte Cytostatikum wirkt in ähnlichen Bedingungen, wie sie auch in-vivo auftreten. Hierdurch wird nicht nur die Wechselwirkung Cytostatikum Carcinomzelle untersucht, sondern auch die Wirkung des Cytostatikums auf die interagierenden Hepatocytenplättchen und Carcinomzellen. Carcinomzellen brauchen für das normale Wachstum die Gegenwart von nicht-entarteten Zellen. Durch diesen Test ist es möglich, sich weitgehend diesem System der gegenseitigen Beeinflussung anzunähern. Diese Annäherung kann jedoch auch durch ein durch Hepatocytenplättchen konditioniertes Medium erfolgen. Dazu wird der gleiche Ansatz wie bei der Kokultur gewählt, auf das direkte Einbringen der Carcinomzellen wird jedoch im ersten Schritt verzichtet. Dadurch wirkt das Cytostatikum direkt auf die Hepatocytenplättchen, die Mediatoren und Metaboliten in das Kulturmedium abgeben, wodurch ein konditioniertes Medium entsteht. Das konditionierte Medium kann eingefroren werden. Die im konditionierten Medium enthaltenen Metaboliten werden dadurch in ihrer Stabilität nicht beeinflußt. In dem konditionierten Medium werden dann -in einem Verfahren nach Beispiel 1- Tumorzellen kultiviert.

Weitere Merkmale, Einzelheiten und Vorteile der Metabolitentestung ergeben sich aus der beigefügten Zeichnung sowie aus der nachfolgenden Beschreibung von verschiedenen vorteilhaften Ausführungsformen der erfindungsgemäßen Metabolitentestung durch vorgeschaltete beziehungsweise integrierte in-vito-Leberpassagen.

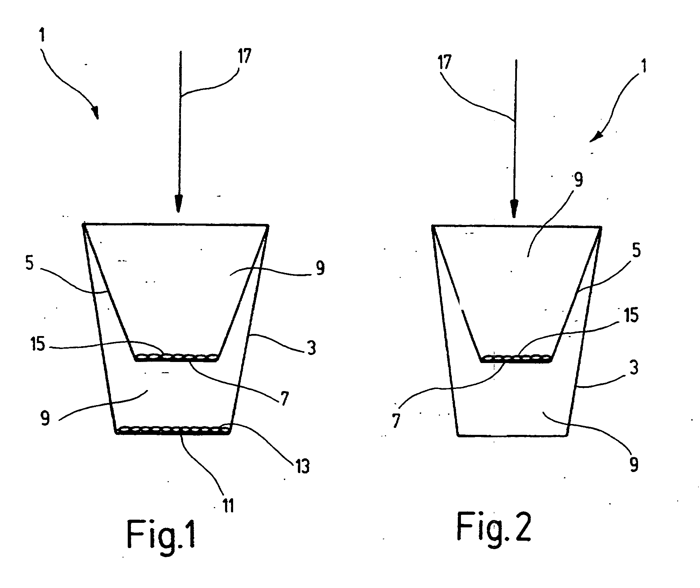

Figur 1 zeigt schematisch eine Ausführungsform der Kokultur. In einem Zwei-Kammersystem 1. Das Zwei-Kammersystem 1 besteht aus einem Kulturgefäß 3, in dem ein Einsatz 5 eingebracht wurde. Das Kulturgefäß 3 und der Einsatz 5 werden durch die metabolitendurchlässige Membran 7 räumlich getrennt, wobei die kultivierten Zellen 11, 15 die Membran 7 nicht passieren können. Das heißt, zwischen dem Kulturgefäß 3 und dem Einsatz 5 ist ein Stoffaustausch in Form von Diffussion durch eine Membran 7 möglich. Die Membran 7 kann so gewählt sein, daß sie entweder nur für bestimmte Ionen selektiv durchlässig ist, also eine qualitative Selektion vornimmt oder sie kann so gewählt sein, daß sie eine Selektion nach quantitativen Parametern vornimmt. Quantitative Parameter können zum Beispiel die Anzahl der.Ladungsträger oder die Größe der Ionen und/oder der Biomoleküle sein.

Das Zwei-Kammersystem 1 kann mit Kulturmedium 9 gefüllt werden, indem das Kulturmedium 9 in den Einsatz 5 eingebracht wird und folgend durch die Membran 7 in das Kulturgefäß 3 gelangt. Durch dieses Verfahren können sich, je nach eingesetztem Medium und Membran, in dem Kulturgefäß 3 und in dem Einsatz 5 Kulturmedien 9 unterschiedlicher Zusammensetzung befinden, da gegebenenfalls nicht alle Bestandteile des Kulturmediums 9 durch die Membran 7 hindurch in das Kulturgefäß 3 diffundieren können. Es kann jedoch auch vorgesehen sein, erst das Kulturgefäß 3 -ohne daß sich bereits ein Einsatz 5 in diesem befindet- mit Kulturmedium 9 aufzufüllen und anschließend den Einsatz 5 in das Kulturgefäß einzubringen, um folgend Kulturmedium 9 auch in den Einsatz 5 zu gießen. Durch dies Form des Befüllens des Zwei-Kammersystems 1 ist gewährleistet, daß in Einsatz 5 und in Kulturgefäß 3 ein Kulturmedium 9 gleicher Zusammensetzung vorhanden ist. Das so behandelte Zwei-Kammersystem 1 wird so lange stehengelassen, bis gewährleistet ist, daß die Membran 7 mit Kulturmedium 9 vollständig durchdrungen ist. Durch diese Zeitdauer ist außerdem gewährleistet, daß sich das Kulturmedium 9 optimal im Kulturgefäß 3 und in dem Einsatz 5 verteilt. Der Aufbau einer Kokultur geschieht dadurch, daß zwei verschiedene Zelltypen in das Zwei-Kammersystem 1 eingebracht werden. Hierzu wird der Einsatz 5 mit einer in der Abbildung nicht dargestellten sterilen Pinzette so weit angehoben, daß eine ebenfalls nicht dargestellte mit Zellen gefüllte Pipette in das Kulturgefäß 3 eingebracht werden kann. Das Einbringen der Pipette in das Kulturgefäß 3 erfolgt so, daß das Pipettenende sich in geringer Entfernung zu der Beschichtung 11 des Bodens des Kulturgefäßes 3 befindet. Die Beschichtung kann aus Collagen bestehen. Es ist jedoch auch möglich, auf eine Beschichtung 11 des Bodens zu verzichten. Auf der Beschichtung 11 des Kulturgefäßes 3 werden Carcinomzellen 13 eingesät. Die Carcinomzellen 13 sind aus Gründen der Anschaulichkeit in Figur 1 sehr stark vergrößert dargestellt. Nach dem Einsäen der Carcinomzellen 13 wird der Einsatz 5 mit der sterilen Pinzette in seine ursprüngliche Position gebracht. Anschließend werden mit einer neuen Pipette Hepatocytenplättchen 15 in den Einsatz 5 so eingebracht, daß sie sich auf der zum Einsatz 5 gewandten Fläche der Membran 7 befinden. Somit befinden sich in dem Zwei-Kammersystem 1 zwei verschiedene Zelltypen, nämlich die Carcinomzellen 13 und die Hepatocytenplättchen 15, die jedoch räumlich durch die Membran 7 getrennt sind. Die Carcinomzellen 15 und die Hepatocytenplättchen 15 befinden sich jedoch durch die Membran 7 und das Medium 9 in einem Stoff- beziehungsweise Diffusionszusammenhang. Metaboliten oder Mediatoren, die die Carcinomzellen 13 in das Medium 9 abgeben, können durch die Membran 7 in den Einsatz 5 diffundieren und so auf die Hepatocytenplättchen 15 wirken. Umgekehrt ist es jedoch auch möglich, daß biologische und chemische Verbindungen, die durch die Hepatocytenplättchen 15 gebildet werden, folgend in das Medium 9 des Einsatzes 5 eindringen und durch die Membran 7 in das Kulturgefäß 3 diffundieren, wo sie direkt auf die Carcinomzellen 13 einwirken können. Nachdem diese Kokultur in dem Zwei-Kammersystem 1 hergestellt wurde, wird das Cytostatikum 17 in das Zwei-Kammersystem 1 so eingebracht, daß es beispielsweise mittels einer nicht dargestellten Pipette in den Einsatz 5 getropft wird. Das in dem Einsatz 5 eingebrachte Cytostatikum 17 verteilt sich in dem Kulturmedium 9 des Einsatzes 5. Nach einer kurzen Zeitspanne trifft das Cytostatikum 17 auf die Hepatocytenplättchen 15 und beginnt mit diesen wechselzuwirken. Im folgenden dringt das Cytostatikum 17 durch die Membran 7 in das Kulturgefäß 3 ein. Die Hepatocytenplättchen 15 werden durch das Cytostatikum 17 in ihrer Stoffwechselaktivität modifiziert, wodurch sie spezifische Metaboliten abgeben können. Diese dringen ebenfalls in das Kulturmedium 9 ein und passieren die Membran 7 und befinden sich folgend im Kulturgefäß 3, wo sie auf die Carcinomzellen 13 einwirken.können. Die Carcinomzellen 13 interagieren mit dem Cytostatikum 17 wie auch mit den Metaboliten der Hepatocytenplättchen 15 und geben daraufhin eigene Stoffwechselprodukte ins Kulturmedium 9 ab. Diese Stoffwechselprodukte können ebenfalls die Membran 7 durchdringen und so direkt auf die Hepatocytenplättchen 15 einwirken. Durch dieses gegenseitige Beeinflussen der unterschiedlichen Zelltypen untereinander und aufgrund des Einwirkens des Cytostatikums 17 auf die verschiedenen Zelltypen in der Kokultur im Zwei-Kammersystem 1 entsteht eine Interaktion, die den physiologischen in-vivo-Prozessen weitgehend angepaßt ist. Bei der dargestellten Kokultur gibt es somit keine Phase der Vorkultur, also eine vorherige Kultivierung ohne Cytostatikum. Dies bedeutet, daß die unterschiedlichen Zelltypen direkt unter dem Einfluß des zugegebenen Cytostatikums 17 kultiviert werden, ohne vorher eine Vorkulturphase zu realisieren. Ein weiterer wichtiger Aspekt einer optimalen Kokultur im Zwei-Kammersystem 1 ist der, daß die Membran 7 und die Beschichtung 11 beziehungsweise der Boden des Kulturgefäßes 3 eben sind. Durch die weitgehend plane Fläche ist gewährleistet, daß eine überschichtung der Zellen während der Kultivierung verhindert wird. Hierdurch ist für die Hepatocytenplättchen 15 und die Carcinomzellen 13 gleichermaßen eine optimale Versorgung über das Kulturmedium 9 gewährleistet, da sich die Zellen gleichmäßig über die angebotene Fläche verteilt haben. Wäre hingegen die Membran 7 oder die Beschichtung 11 beziehungsweise der Boden so gestaltet, daß sie Vertiefungen aufweisen, würden sich die Zellen dort ansammeln und überschichtet werden. Dadurch wäre eine optimale Versorgung über das Kulturmedium 9 nicht mehr gewährleistet und die Zellen würden natürlich absterben, wodurch verschiedene Toxine freigesetzt würden, die wiederum ein verstärktes Absterben der Zellen im Zwei-Kammersystem 1 induzieren würden. Nachfolgende Messungen würden dadurch verfälscht.

Mittels des in Figur 1 dargestellten Zwei-Kammersystems 1 läuft das erfindungsgemäße Verfahren zur Bestimmung der Aktivität eines Cytostatikums wie folgt ab. Nach Isolierung und gegebenenfalls erfolgendem mechanischen Zerkleinern eines Tumormaterials wird dieses in einer der erfindungsgemäßen Tumordissoziierungslösungen zur Einzelzellsuspension dissoziiert. Nach Abtrennung der Einzelzellen von der Tumordissoziierungslösung werden diese entweder in das Gefäß 3 oder in das Gefäß 5 eingebracht und in das jeweils andere Gefäß zum Beispiel Hepatocytenplättchen 15 eingebracht. Es findet dann die vorstehend beschriebene Kokultivierung unter Einsatz eines ebenfalls in das Zwei-Kammersystem 1 eingebrachten Cytostatikums statt. Anschließend wird das Kulturmedium 9 abgenommen und in das Zwei-Kammersystem 1, insbesondere dessen Einsatz 5 und/oder in das Kulturgefäß 3 Tumorextraktionspuffer, insbesondere Lysepuffer, eingebracht. Sodann kann in dem Zwei-Kammersystem 1, das heißt in dem Kulturgefäß 3 oder dem Einsatz 5, vorzugsweise in dem Kulturgefäß 3, die luminometrische ATP-Bestimmung mittels Luciferin-Luciferase durchgeführt werden.

In der Figur 2 ist ein Experimentalansatz dargestellt, mit dem ein konditioniertes Medium hergestellt wird. Das konditionierte Medium wird in eidem Zwei-Kammersystem 1 gewonnen, welches dem in Figur 1 beschriebenen Zwei-Kammersystem 1 entspricht. Deshalb werden im folgenden die identischen Bezugsziffern für gleiche Bestandteile des Zwei-Kammersystems 1 verwandt. Das Zwei-Kammersystem 1 zur Herstellung von konditioniertem Medium besteht wiederum aus einem Kulturgefäß 3 und einem dort eingebrachten Einsatz 5. Kulturgefäß 3 und Einsatz 5 sind über eine Membran 7 miteinander verbunden. Kulturgefäß 3 und Einsatz 5 sind mit Kulturmedium 9 angefüllt. In dem Einsatz 5 wird auf der Membran 7 eine Kultur von Hepatocytenplättchen 15 aufgebracht. Aus Gründen der Anschaulichkeit sind die Hepatocytenplättchen 15 stark vergrößert dargestellt. Durch den Aufbau des Zwei-Kammersystems 1 ist, wie bereits in Figur 1 dargestellt, gewährleistet, daß ein Stoffaustausch zwischen Kulturgefäß 3 und Einsatz 5 über die Membran 7 stattfinden kann, wodurch gewährleistet ist, daß das Kulturmedium 9 an jedem Punkt- des Zwei-Kammersystems 1 eine ähnliche Zusammensetzung in bezug auf im Kulturmedium 9 löslichen kleinen Moleküle aufweist. Dieser Status der weitgehend gleichmäßigen Verteilung kann dadurch optimiert werden, daß das Zwei-Kammersystem 1 so bewegt wird, daß die Kultivierung der in diesem System eingebrachten Zellen nicht negativ beeinflußt wird. Nach Aussaat der Hepatocytenplättchen 15 auf die Membran 7 wird das Cytostatikum 17 in das Kulturmedium 9 eingebracht. Das Cytostatikum 17 beginnt daraufhin, sich in dem Kulturmedium 9 auszubreiten und trifft auf die Hepatocytenplättchen 15, wodurch diese spezifische Metaboliten an das Kulturmedium 9 abgeben, die durch die Membran 7 in das Kulturgefäß 3 diffundieren. Nach einem gewissen Zeitraum befinden sich deshalb im gesamten Kulturmedium 9 die Metaboliten der Hepatocytenplättchen 15, die zum einen durch das normale Wachstum und die normalen Stoffwechselvorgänge der Zellen gebildet werden, wie auch die Metaboliten, die von den Hepatocytenplättchen 15 unter Einfluß des Cytostatikums 17 synthetisiert werden. Diese Metaboliten wirken nun zurück auf die Hepatocytenplättchen 15, die dadurch in ihrer Stoffwechselleistung und in den physiologischen Vorgängen verändert werden. Sämtliche daraus resultierenden biochemischen Verbindungen, die von den Hepatocytenplättchen 15 ins Kulturmedium 9 abgegeben werden, führen dazu, daß das Kulturmedium 9 konditioniert wird.

Das konditionierte Medium kann anschließend abgenommen werden, um in diesem verschiedene Tumorzellen zu kultivieren. Die experimentellen Erfahrungen zeigen, daß es möglich ist, das konditionierte Medium einzufrieren, weil dadurch die Stabilität der Metaboliten nicht negativ beeinflußt wird.

### Literatur

Andreotti, P. E., Thornthwaite J. T., Morse, I. S., ATP Tumor Chemosensitivity Assay. In: Stanley, P. E., Kricka, L. J. (eds.) Bioluminescence and Chemiluminescence: Current Status. Chichester, J. Wiley & Sons, Seite 417-420 (1991)

Andreotti, P. E., Chemosensitivity testing of human tumors using a microplate adenosine triphosphate luminescence assay: clinical correlation for cisplatin resistance of ovarian carcinoma. *Cancer* Re*search* 55, Seite 5276-5282 (1995)

Bellamy, W. T., Prediction of response to drug therapy of cancer. A review of in vitro assays. Drugs 44, 5, Seite 690-708 (1992)

Cree, I. A., Correlation of the clinical response to chemotherapy in breast cancer with ex vivo chemosensitivity. *Anti-Cancer Drugs* 7, Seite 630-635 (1996)

Hanauske, A. R. and Hoff, v. D., The value of the human tumor clonong assay in ovarian cancer. Clin. Obstet. Gynecol. 29, 3, Seiten 638-644 (1986)

Hunter, E. M., Sutherland, L. A., Cree, I. A., Heterogeneity od chemosensitivity in human breast carcinoma: use of an adenosine triphosphate (ATP) chemiluminescence assay. Europ. J. Surg. Onco., 19, Seite 242-249 (1993)

Kurbacher, C. M., Chemosenibilitätstestung in der gynäkologischen Onkologie: Erfahrungen mit einem ATP-Biolumineszenzassay. *Geburtsh*. *und Frauenheilk*. 56, Seite 70-78 (1996 a)

Kurbacher, C. M., Heterogeneity of in vitro chemosensititvity in perioperative breast cancer cells to mitoxantrone versus doxorubicin evaluated by a microplate ATP bioluminescence assay. *Breast Cancer Research* and *Treatment* 41, Seite 161-170 (1996 b)

Pieters, R., In vito drug sensitivity of cells from children with leukaemia using the MTT assay with improved culture conditions. Blood 76, Seite 2327-2336 (1990)

Satori, C., ATP tumor chemosensitivity assay (ATP-TCA) in gynaecological oncology. *Eur. J. Clin. Chem. Clin. Biochem*. 35, Seite 647-736 (1997)

Sevin, B. U., Chemosensitivity testing in ovarian cancer. Cancer 71 (4), Seite 1613-1620 (1993)

Volm, M., Prediction of the clinical chemmotherapeutic response of stage III lung adenocarcinoma patients by an in vitro short term test. Br. J. Cancer, 57, Seite 198-200 (1988)

Weisenthal, L. and Kern, D. , Prediction of drug restistance in cancer Therapy: The Kern and DiSC assays. Oncology 5, Seite 93-103 (1991)

## Patentansprüche

1. Verfahren zum Bestimmen der Aktivität mindestens eines Cytostatikums gegenüber isoliertem Tumormaterial in Form von Tumorgewebe oder malignem Ergußmaterial, wobei das Verfahren die folgenden Schritte umfaßt:
a) das Zentrifugieren des Ergussmaterials zur Anreicherung der darin enthaltenen Zellen oder das mechanische Zerkleinern des Tumorgewebes,
b) das enzymatische Dissoziieren des Tumormaterials, wobei das Tumormaterial in einer Dissoziierungslösung enthaltend 500000 bis 730000 units/100 ml Collagenase, 3500 bis 6500 units/100 ml DNAse, 0,2 bis 1,6 Gew.-% BSA sowie gegebenenfalls eine Protease, Carbohydrase und/oder Lipase über einen Zeitraum von 10 bis 120 Minuten inkubiert wird, der ausreicht, eine Suspension von Einzelzellen herzustellen,
c) die Zugabe des zu bestimmenden mindestens einen Cytostatikums zu der erhaltenen Suspension,
d) das Kultivieren der Suspension in einem Kulturmedium in Gegenwart des Cytostatikums,
e) das Auf schließen der kultivierten Einzelzellen durch Tumorextraktionslösung unter Zugabe eines Lumineszenz- und/oder eines photosensibilisierten Farbstoffes und
f) das Messen der ATP-Aktivität anhand der auftretenden Lumineszenz.

2. Verfahren nach Anspruch 1, wobei das Tumormaterial Material aus minimalinvasiven und/oder microinvasiven Eingriffen ist.

3. Verfahren nach Anspruch 2, wobei das Material aus minimalinvasiven und/oder microinvasiven Eingriffen Biopsiematerial, Pleural- und/oder Ascitespunktatmaterial ist.

4. Verfahren nach Anspruch 1, wobei die Protease Pronase E, die Carbohydrase Hyaluronidase und die Lipase Pankreatin ist.

5. Verfahren nach Anspruch 1, wobei die Dissoziierungslösung Pankreatin in einer Menge von 0,03 bis 0,07 Ges.-%, Pronase E in einer Menge von 150 bis 345 units/100 ml und/oder Hyaluronidase in einer Menge von 300000 bis 520000 units/100 ml enthält.

6. Verfahren nach einem der vorhergehenden Ansprüche, wobei die Kultivierung der Einzelzellen über einen Zeitraum von 3 bis 7 Tagen durchgeführt wird.

7. Verfahren nach einem der vorhergehenden Ansprüche, wobei die Kultivierung der Einzelzellen in einem konditionierten Kulturmedium erfolgt, das erhalten wird, indem das Kulturmedium vor Zugabe zu den Einzelzellen mit nicht-entarteten Zellen inkubiert wird.

8. Verfahren nach Anspruch 6, wobei die Kultivierung der Einzelzellen als Kokultivierung erfolgt.

9. Verfahren nach Anspruch 8, wobei die Kokultivierung durch die gleichzeitige Kultivierung von Einzelzellen und nicht-entarteten Zellen in einen den Metabolitenaustausch untereinander ermöglichenden Kulturmedium erfolgt.

10. Verfahren nach Anspruch 8, wobei die Kokultivierung in einer Microplatte mit einer räumlichen Trennung oder ohne räumliche Trennung der Einzelzellen und der nicht-entarteten Zellen erfolgt.

11. Verfahren nach Anspruch 10, wobei die räumliche Trennung durch eine für Metaboliten durchlässige Membran oder einen Filter erfolgt.

12. Verfahren nach einem der Ansprüche 7 bis 11, wobei die nicht-entarteten Zellen Fibroblasten und/oder Hepatocyten sind.

13. Verfahren nach einem der vorhergehenden Ansprüche, wobei die Kultivierung der Einzelzellen und das Messen der ATP-Aktivität in Microplatten, die mit Proteinen der extrazellulären Matrix beschichtet sind, erfolgt.

14. Verfahren nach Anspruch 13, wobei die Proteine der extrazellulären Matrix Collagen und/oder Fibronectin sind.

15. Verfahren nach einem der vorhergehenden Ansprüche, wobei der Lumineszenz-Farbstoff Luciferin-Luciferase ist.

16. Verfahren nach einem der vorhergehenden Ansprüche, wobei das gesamte Verfahren über einen Zeitraum von 20 bis 500 Minuten durchgeführt wird, insbesondere 20 bis 240 Minuten.

17. Verfahren nach einem der vorhergehenden Ansprüche, wobei die Kultivierung und/oder Kokultivierung der Einzelzellen bei ansteigender Temperatur erfolgt, insbesondere über einen Temperaturbereich von 37°C bis 41°C.

18. Verfahren zur luminometrischen ATP-Bestimmung von isoliertem potentiell entartete Zellen enthaltenden Material insbesondere Tumormaterial in Form von Tumorgewebe oder malignem Ergußmaterial, wobei das Verfahren die folgenden Schritte umfasst:
a) das Zentrifugieren des Ergussmaterials zur Anreicherung der darin erhaltenen freien Zellen oder das mechanische Zerkleinern des Tumorgewebes,
b) das enzymatische Dissoziieren des Tumormaterials, wobei das Tumormaterial in einer Dissoziierungslösung enthaltend 500000 bis 730000 units/100 ml Collagenase, 3500 bis 6500 units/100 ml DNAse und 0,2 bis 1,6 Gew.-% BSA und gegebenenfalls eine Dissoziierungslösung enthaltend eine Protease, Carbohydrase und/oder Lipase über einen Zeitraum von 10 bis 120 Minuten inkubiert wird, der ausreicht, eine Suspension von Einzelzellen herzustellen,
c) das Aufschließen der Einzelzellen durch Tumorextraktionslösung unter Zugabe eines Lumineszenz- und/oder eines photosensibilisierten Farbstoffes und
d) das Messen der ATP-Aktivität anhand der auftretenden Lumineszenz.

19. Verfahren nach einem der vorhergehenden Ansprüche, wobei die Einzelzellen vor dem Aufschließen immunomagnetisch aufgereinigt werden, um eine Anreicherung von Tumorzellen zu erreichen.

## Claims

1. Method for determining the activity of at least one cytostatic agent in relation to isolated tumour material in the form of tumour tissue or malignant effusion material, the method comprising the following steps:
a) centrifuging the effusion material to concentrate the cells contained therein, or mechanically reducing the tumour tissue,
b) the enzymatic dissociation of the tumour material, the tumour material being incubated in a dissociation solution containing 500000 to 730000 units/100 ml collagenase, 3500 to 6500 units/100 ml DNAse, 0.2 to 1.6 wt-% BSA as well as possibly a protease, carbohydrase and/or lipase, over a period of 10 to 120 minutes which is sufficient to produce a suspension of single cells,
c) the addition of the at least one cytostatic agent to be determined to the suspension obtained,
d) the cultivation of the suspension in a culture medium in the presence of the cytostatic agent,
e) breaking up the cultivated single cells by tumour extract solution with the addition of a luminescent dye and/or a photosensitized dye, and
f) measuring the ATP activity on the basis of the luminescence occurring.

2. Method according to claim 1, wherein, the tumour material is material from minimally invasive and/or microinvasive operations.

3. Method according to claim 2, wherein the material from minimally invasive and/or microinvasive operations is biopsy material, pleural and/or ascites punctate material.

4. Method according to claim 1, wherein the protease is pronase E, the carbohydrase is hyaluronidase and the lipase is pancreatine.

5. Method according to claim 1, wherein the dissociation solution contains pancreatine in an amount of between 0.03 and 0.07 wt-%, pronase E in an amount of between 150 and 345 units/100 ml and/or hyaluronidase in an amount of between 300000 and 520000 units/100 ml.

6. Method according to one of the preceding claims, wherein the cultivation of the single cells is carried out over a period of 3 to 7 days.

7. Method according to one of the preceding claims, wherein the cultivation of the individual cells takes place in a conditioned culture medium which is obtained by the culture medium being incubated with non-degenerate cells before being added to the single cells.

8. Method according to claim 6, wherein the cultivation of the single cells takes place as co-cultivation.

9. Method according to claim 8, wherein the co-cultivation takes place through the simultaneous cultivation of single cells and non-degenerate cells in a culture medium which makes possible the exchange of metabolites between these cells.

10. Method according to claim 8, wherein the co-cultivation takes place in a microplate with spatial separation or without spatial separation of the single cells and the non-degenerate cells.

11. Method according to claim 10, wherein the spatial separation takes place through a membrane which is permeable by metabolites or through a filter.

12. Method according to one of claims 7 to 11, wherein the non-degenerate cells are fibroblasts and/or hepatocytes.

13. Method according to one of the preceding claims, wherein the cultivation of the single cells and the measuring of the ATP activity takes place in microplates which are coated with proteins of the extracellular matrix.

14. Method according to claim 13, wherein the proteins of the extracellular matrix are collagen and/or fibronectin.

15. Method according to one of the preceding claims, wherein the luminescent dye is luciferin-luciferase.

16. Method according to one of the preceding claims, wherein the entire process is carried out over a period of 20 to 500 minutes, especially 20 to 240 minutes.

17. Method according to one of the preceding claims, wherein the cultivation and/or co-cultivation of the single cells takes place at a rising temperature, especially over a temperature range from 37°C to 41°C.

18. Method for the luminometric ATP-determination of isolated material potentially containing degenerate cells, especially tumour material in the form of tumour tissue or malignant effusion material, the method comprising the following steps:
a) centrifuging the effusion material to concentrate the free cells contained therein, or mechanically reducing the tumour tissue,
b) the enzymatic dissociation of the tumour material, the tumour material being incubated in a dissociation solution containing 500000 to 730000 units/100 ml collagenase, 3500 to 6500 units/100 ml DNAse and 0.2 to 1.6 wt-% BSA and possibly a dissociation solution containing a protease, carbohydrase and/or lipase over a period of 10 to 120 minutes, which is sufficient to produce a suspension of single cells.
c) breaking up the single cells by tumour extract solution with the addition of a luminescent dye and/or a photosensitized dye, and
d) measuring the ATP activity on the basis of the luminescence occurring.

19. Method according to one of the preceding claims, wherein the single cells are immunomagnetically purified before being broken up, in order to achieve a concentration of tumour cells.

## Revendications

1. Procédé pour la détermination de l'activité d'au moins un cytostatique vis-à-vis d'une matière tumorale isolée sous forme de tissu tumoral ou de matière d'épanchement de nature maligne, ce procédé comprenant les étapes suivantes:
a) la centrifugation de la matière d'épanchement pour l'enrichissement des cellules qu'elle contient ou la fragmentation mécanique du tissu tumoral,
b) la dissociation enzymatique de la matière tumorale, la matière tumorale étant incubée dans une solution de dissociation contenant 500.000 à 730.000 unités/100 ml de collagénase, 3.500 à 6.500 unités/ml de ADNase, 0,2 à 1,6 % en poids d'ABS ainsi que, le cas échéant, une protéase, une carbohydrase et/ou une lipase pendant une durée de 10 à 120 minutes, qui suffit pour préparer une suspension de cellules individuelles.
c) l'addition du au moins un cytostatique, dont l'activité est à déterminer, à la suspension obtenue,
d) la culture de la suspension dans un milieu de culture en présence du cytostatique,
e) le traitement des cellules individuelles cultivées par une solution d'extraction de tumeur avec addition d'un colorant de luminescence et/ou d'un colorant photosensibilisant, et
f) la mesure de l'activité ATP d'après la luminescence produite.

2. Procédé selon la revendication 1,
dans lequel la matière tumorale provient d'interventions invasives minimales et/ou micro-invasives.

3. Procédé selon la revendication 2,
dans lequel la matière résultant d'interventions invasives minimales et/ou micro-invasives est une matière de biopsie, pleurale et/ou de ponction d'ascite.

4. Procédé selon la revendication 1,
dans lequel la protéase est la pronase E, la carbohydrase est l'hyaluronidase et la lipase est la pancréatine.

5. Procédé selon la revendication 1,
dans lequel la solution de dissociation contient de la pancréatine dans une proportion de 0,03 à 0,07 % en poids, de la pronase E dans une proportion de 150 à 345 unités/100 ml et/ou de la hyaluronidase dans une proportion de 300.000 à 520.000 unités/100 ml.

6. Procédé selon l'une quelconque des revendications précédentes,
dans lequel la culture des cellules individuelles est effectuée sur une périodes de 3 à 7 jours.

7. Procédé selon l'une quelconque des revendications précédentes,
dans lequel la culture de cellules individuelles a lieu dans un milieu de culture conditionné, qui est obtenu en incubant le milieu de culture, avant l'addition aux cellules individuelles, avec des cellules non dégénérées.

8. Procédé selon la revendication 6,
dans lequel la culture des cellules individuelles a lieu sous forme de co-culture.

9. Procédé selon la revendication 8,
dans lequel la co-culture a lieu par la culture simultanée des cellules individuelles et de cellules non dégénérées dans un milieu de culture permettant l'échange des métabolites entre eux.

10. Procédé selon la revendication 8,
dans lequel la co-culture a lieu dans une micro-plaque avec une séparation spatiale ou sans séparation spatiale des cellules indidivuelles et des cellules non dégénérées.

11. Procédé selon la revendication 10,
dans lequel la séparation spatiale a lieu au moyen d'une membrane perméable pour les métabolites ou d'un filtre.

12. Procédé selon l'une des revendications 7 à 11,
dans lequel les cellules non dégénérées sont des fibroblastes et/ou des hépatocytes.

13. Procédé selon l'une quelconque des revendications précédentes,
dans lequel la culture des cellules individuelles et la mesure de l'activité ATP ont lieu dans des micro-plaques qui sont revêtues avec des protéines de la matrice extra-cellulaire.

14. Procédé selon la revendication 13,
dans lequel les protéines de la matrice extra-cellulaire sont le collagène et/ou la fibronectine.

15. Procédé selon l'une quelconque des revendications précédentes,
dans lequel le colorant de luminescence est la luciférine-luciférase.

16. Procédé selon l'une quelconque des revendications précédentes,
dans lequel l'ensemble du procédé est effectué sur une période de 20 à 500 minutes, en particulier de 20 à 240 minutes.

17. Procédé selon l'une quelconque des revendications précédentes,
dans lequel la culture et/ou la co-culture des cellules individuelles a (ont) lieu à une température croissante, en particulier sur une plage de température de 37 °C à 41 °C.

18. Procédé pour la détermination d'ATP luminométrique de matière contenant des cellules potentiellement dégénérées, isolée, en particulier une matière tumorale, sous forme de tissu tumoral ou de matière d'épanchement de nature maligne, ce procédé comportant les étapes suivantes :
a) la centrifugation de la matière d'épanchement pour l'enrichissement des cellules qu'elle contient ou la fragmentation mécanique du tissu tumoral,
b) la dissociation enzymatique de la matière tumorale, la matière tumorale étant incubée dans une solution de dissociation contenant 500.000 à 730.000 unités/100 ml de collagénase, 3.500 à 6.500 unités/ml de ADNase, 0,2 à 1,6 % en poids d'ABS ainsi que, le cas échéant, une solution de dissociation contenant une protéase, une carbohydrase et/ou une lipase pendant une durée de 10 à 120 minutes, qui suffit pour préparer une suspension de cellules individuelles,
c) le traitement des cellules individuelles par une solution d'extraction de tumeur avec addition d'un colorant de luminescence et/ou d'un colorant photosensibilisant, et
d) la mesure de l'activité ATP d'après la luminescence produite.

19. Procédé selon l'une quelconque des revendications précédentes, dans lequel les cellules individuelles sont purifiées de manière immuno-magnétique avant le traitement, pour obtenir un enrichissement de cellules tumorales.
